# EUROPÄISCHE PATENTSCHRIFT

(11) **EP 1 037 593 B1**
(45) Veröffentlichungstag und Bekanntmachung des Hinweises auf die Patenterteilung: **04.02.2004**
(21) Anmeldenummer: 99944624.8
(22) Anmeldetag: 08.09.1999
(51) Int. Cl.: A61K 7/13

(54) **MITTEL UND VERFAHREN ZUR FÄRBUNG VON FASERN**
AGENT AND METHOD FOR DYEING FIBRES
SUBSTANCE ET PROCEDE DE TEINTURE DE FIBRES

(30) Priorität: 13.10.1998 DE 19847192
(43) Veröffentlichungstag der Anmeldung: 27.09.2000
(73) Patentinhaber: Wella Aktiengesellschaft, 64274 Darmstadt (DE)
(72) Erfinder: BRAUN, Hans-Jürgen, Dr., CH-3182 Überstorf (CH); SEMADENI, Pascal André, Dr., CH-1792 Cordast (CH)
(86) Internationale Anmeldenummer: PCT/EP1999/006601
(87) Internationale Veröffentlichungsnummer: WO 2000/021496

(56) Entgegenhaltungen:
- DE-A- 2 737 291
- DE-A- 2 827 658
- DE-A- 4 205 329
- DE-A- 4 404 563
- FR-A- 1 506 945
- FR-A- 1 584 965
- FR-A- 2 211 210
- US-A- 5 135 543

## Beschreibung

Gegenstand der Erfindung ist ein Mittel zur Färbung von Fasern, insbesondere keratinischen Fasem, mit einem Gehalt an Nitrophenylaminen sowie ein Verfahren zum Färben von Haaren unter Verwendung dieses Mittels.

Insbesondere für die Haarfärbung haben Nitrofarbstoffe eine wesentliche Bedeutung erlangt. Sie sind in Oxidationshaarfärbemitteln ein wichtiger Bestandteil zur Abrundung des Farbergebnisses und zur Erzeugung von modischen Farbnuancen. Durch Kombination von gelben, roten und blauen Nitrofarbstoffen lassen sich jedoch auch Färbemittel herstellen, die ohne Zusatz von Oxidationsmitteln Haare in natürlichen bis modischen Tönen zu färben vermögen.

Von besonderer Bedeutung sind hierbei direktziehende gelbe Nitrofarbstoffe, welche das Haar in einem intensiven Zitronengelb einfärben, das möglichst frei von Rotanteilen sein soll. Weiterhin müssen Nitrofarbstoffe noch eine ganze Reihe von zusätzlichen Anforderungen erfüllen. So müssen sie in toxikologischer und dermatologischer Hinsicht unbedenklich sein und die Erzielung von Färbungen in der gewünschten Intensität ermöglichen, was unter anderem auch eine ausreichende Wasserlöslichkeit voraussetzt. Außerdem wird für die erzielten Färbungen eine gute Lichtechtheit, Reibeechtheit, Dauerwellechtheit und Schweißechtheit gefordert.

Andererseits dürfen die Farbstoffe bei der Nachbehandlung oder bei der Haarwäsche nicht zu leicht wieder ausgewaschen werden. Ihre Verwendung in Oxidationshaarfärbemitteln setzt weiter voraus, daß sie in Anwesenheit von Reduktionsmitteln und Oxidationsmitteln, insbesondere in alkalischer Lösung, stabil sind.

Es wurde nun überraschend gefunden, daß bestimmte 4-Nitro-phenylamine eine gute Wasserlöslichkeit besitzen und eine intensiv leuchtend gelbe Färbung ermöglichen sowie eine hervorragende Lichtechtheit, Reibeechtheit, Dauerwellechtheit und Schweißechtheit aufweisen.

Die vorliegende Erfindung betrifft daher Mittel zum Färben von Fasern, insbesondere Keratinfasem, wie zum Beispiel menschlichen Haaren, welche dadurch gekennzeichnet sind, daß sie mindestens eine Verbindung aus der Gruppe bestehend aus N-(2-Methoxyethyl)-4-nitrophenylamin, N-(2-Methoxyethyl)-2-methyl-4-nitro-phenylamin, N-(3-Methoxypropyl)-4-nitro-phenylamin und N-(3-Methoxypropyl)-2-methyl-4-nitro-phenytamin enthalten.

Die erfindungsgemäßen Farbstoffe werden in den erfindungsgemäßen Färbemitteln vorzugsweise in einer Gesamtmenge von 0,01 bis 10 Gewichtsprozent, insbesondere in einer Gesamtmenge von 0,1 bis 5 Gewichtsprozent, eingesetzt.

Die erfindungsgemäßen Farbstoffe sind aus verfügbaren Vorstufen leicht herstellbar und besitzen nicht nur eine gute Wasserlöslichkeit, sondern auch ein sehr gutes Aufziehverhalten aus anionischen, kationischen, amphoteren oder nichtionogenen Trägermassen. Die erhaltenen Färbungen besitzen eine hohe Beständigkeit gegenüber Auswaschen, Schweiß und Lichtbestrahlung sowie gegenüber Basen wie Ammoniak und Säuren wie Phosphorsäure, oder Reduktionsmitteln wie Ascorbinsäure oder Natriumsulfit, wodurch ein Einsatz in oxidativen Färbemitteln erheblich erleichtert wird. Die erfindungsgemäßen Färbemittel weisen zudem eine hohe Lagerstabilität auf und ermöglichen intensiv leuchtende gelbe Farbtöne, mit Variationsmöglichkeiten in allen Abstufungen und Nuancen von einem helle Zitronengelb bis zu einem intensiven Dunkelgelb.

Die erfindungsgemäßen Färbemittel können sowohl ohne als auch unter Zugabe eines Oxidationsmittels angewendet werden wobei im letzteren Fall übliche Entwicklersubstanzen und Kupplersubstanzen zugesetzt werden.

Wird das Färbemittel ohne Oxidationsmittelzugabe angewendet, so kann es neben den erfindungsgemäßen Farbstoffen zusätzlich noch weitere bekannte direktfärbende Farbstoffe aus der Gruppe bestehend aus Nitrofarbstoffen, Azofarbstoffen, Anthrachinonfarbstoffen und Triphenylmethanfarbstoffen, alleine oder im Gemisch miteinander, enthalten.

Beispiele für geeignete direktziehende Farbstoffe sind 1,4-Bis[(2-hydroxyethyl)amino]-2-nitrobenzol, 1-(2-Hydroxyethyl)amino-2-nitro-4-[di(2-hydroxyethyl)ämino]-benzol (HC Blue No. 2), 1-Amino-3-methyl-4-[(2-hydroxyethyl)amino]-6-nitrobenzol (HC Violet No. 1), 4-[Ethyl-(2-hydroxyethyl)amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxypropyl)amino]-4-[methyl-(2-hydroxyethyl)amino]-2-nitrobenzol (HC Blue No. 10), 1-[(2,3-Dihydroxypropyl)amino]-4-[ethyl-(2-hydroxyethyl)amino]-2-nitrobenzolhydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxypropyl)amino]-2-nitrobenzol (HC Blue No. 6), 2-((4-Amino-2-nitrophenyl)amino)-5-dimethylamino-benzoesäure (HC Blue No. 13), 1-Amino-4-[(2-hydroxyethyl)amino]-2-nitrobenzol (HC Red No. 7), 2-Amino-4,6-dinitro-phenol, 4-Amino-2-nitro-diphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-Amino-3-nitrophenol, 4-[(2-Hydroxyethyl)amino]-3-nitrophenol, 1-[(2-Aminoethyl)amino]-4-(2-hydroxyethoxy)-2-nitrobenzol (HC Orange No. 2), 4-(2,3-Dihydroxypropoxy)-1-[(2-hydroxyethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino]-2-nitrobenzol (HC Red No. 11), 2-[(2-Hydroxyethyl)amino]-4,6-dinitrophenol, 4-Ethylamino-3-nitrobenzoesäure, 2-[(4-Amino-2-nitrophenyl)amino]-benzoesäure, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitropyridin, 1,2,3,4-Tetrahydro-6-nitrochinoxalin, 7-Amino-3,4-dihydro-6-nitro-2H-1,4-benzoxazin (HC Red No. 14), 1-Amino-2-[(2-hydroxyethyl)amino]-5-nitrobenzol (HC Yellow No. 5), 1-(2-Hydroxyethoxy)-2-[(2-hydroxyethyl)amino]-5-nitrobenzol (HC Yellow No. 4), 1'-[(2-Hydroxyethyl)amino]-2-nitrobenzol (HC Yellow No. 2), 2-[(2-Hydroxyethyl)amino]-1-methoxy-5-nitrobenzol, 2-Amino-3-nitrophenol, 1-(2-Hydroxyethoxy)-3-methylamino-4-nitrobenzol, 2,3-(Dihydroxypropoxy)-3-methylamino-4-nitrobenzol, 2-[(2-Hydroxyethyl)amino]-5-nitrophenol (HC Yellow No. 11), 3-[(2-Aminoethyl)amino]-1-methoxy-4-nitrobenzol-hydrochlorid (HC Yellow No.9), 1-[(2-Ureidoethyl)amino]-4-nitrobenzol, 4-[(2,3-Dihydroxypropyl)amino]-3-nitro-1-trifluormethyl-benzol (HC Yellow No. 6), 1-Chlor-2,4-bis[(2-hydroxyethyl)amino]-5-nitrobenzol (HC Yellow No. 10), 4-[(2-Hydroxyethyl)amino]-3-nitro-1-methylbenzol, 1-Chlor-4-[(2-hydroxyethyl)amino]-3-nitrobenzol (HC Yellow No. 12), 4-[(2-Hydroxyethyl)amino]-3-nitro-1-trifluormethyl-benzol (HC Yellow No. 13), 4-[(2-Hydroxyethyl)amino]-3-nitro-benzonitril (HC Yellow No. 14), 4-[(2-Hydroxyethyl)aimino]-3-nitro-benzamid (HC Yellow No. 15), 1,4-Di[(2,3-dihydroxypropyl)amino]-9,10-anthrachinon, 1-[(2-Hydroxyethyl)amino]-4-methylamino-9,10-anthrachinon (Cl61505, Disperse Blue No. 3), 2-[(2-Aminoethyl)amino]-9,10-anthrachinon (HC Orange No. 5), 1-Hydroxy-4-[(4-methyl-2-sulfophenyl)amino]-9,10-anthrachinon, 1-[(3-Aminopropyl)amino]-4-methylamino-9,10-anthrachinon (HC Blue No. 8), 1-[(3-Aminopropyl)amino]-9,10-anthrachinon (HC Red No. 8), 1,4-Diamino-2-methoxy-9,10-anthrachinon (C162015, Disperse Red No. 11, Solvent Violet No. 26), 1,4-Dihydroxy-5,8-bis[(2-hydroxyethyl)amino]-9,10-anthrachinon (CI62500, Disperse Blue No. 7, Solvent Blue No. 69), 9-(Dimethylamino)-benzo[a]phenoxazin-7-ium-chlorid (Cl51175; Basic Blue No. 6), Di[4-(diethylamino)phenyl][4-(ethylamino)naphthyl]carbeniumchlorid (Cl42595; Basic Blue No. 7), 3,7-Di(dimethylamino)phenothiazin-5-ium-chlorid (CI52015; Basic Blue No. 9), Di[4-(dimethylamino)phenyl][4-(phenylamino)naphthyl]carbenium-chlorid (Cl44045; Basic Blue No. 26), 2-[(4-(Ethyl(2-hydroxyethyl)amino)phenyl)azo]-6-methoxy-3-methyl-, benzothiazolium-methylsulfat (CI11154; Basic Blue No. 41), 8-Amino-2-brom-5-hydroxy-4-imino-6-[(3-(trimethylammonio)phenyl)amino]-1(4H)-naphthalinon-chlorid (CI56059; Basic Blue No. 99), Bis[4-(dimethylamino)phenyl][4-(methylamino)phenyl]carbenium-chlorid (CI42535; Basic Violet No. 1), Tris[4-(dimethylamino)phenyl]carbeniumchlorid (CI42555; Basic Violet No. 3), 2-[3,6-(Diethylamino)dibenzopyranium-9-yl]-benzoesäure-chlorid (CI45170; Basic Violet No. 10), Di(4-aminophenyl)(4-amino-3-methylphenyl)carbenium-chlorid (CI42510; Basic Violet No. 14), 1,3-Bis[(2,4-diamino-5-methylphenyl)azo]-3-methylbenzol (CI21010; Basic Brown No. 4), 1-[(4-Aminophenyl)azo]-7-(trimethylammonio)-2-naphthol-chlorid (Cl12250; Basic Brown No. 16), 1-[(4-Amino-2-nitrophenyl)azo]-7-(trimethylammonio)-2-naphthol-chlorid (Basic Brown No. 17), 1-[(4-Amino-3-nitrophenyl)azo]-7-(trimethylammonio)-2-naphthol-chlorid (CI12251; Basic Brown No. 17), 3,7-Diamino-2,8-dimethyl-5-phenylphenazinium-chlorid (CI50240; Basic Red No. 2), 1,4-Dimethyl-5-[(4-(dimethylamino)phenyl)azo]-1,2,4-triazolium-chlorid (Cl11055; Basic Red No. 22), 2-Hydroxy-1-[(2-methoxyphenyl)azo]-7-(trimethylammonio)-naphthalin-chlorid (CI12245; Basic Red No. 76), 2-[2-((2,4-Dimethoxyphenyl)amino)ethenyl]-1,3,3-trimethyl-3H-indol-1-ium-chlorid (CI48055; Basic Yellow No. 11), 3-Methyl-1-phenyl-4-[(3-(trimethylammonio)phenyl)azo]-pyrazol-5-on-chlorid (CI12719; Basic Yellow No. 57), Bis[4-(diethylamino)phenyl]phenylcarbenium-hydrogensulfat (1:1) (CI42040; Basic Green No. 1), 1-[Di(2-hydroxyethyl)amino]-3-methyl-4-[(4-nitrophenyl)azo]-benzol (Cl11210, Disperse Red No. 17), 4-[(4-Aminophenyl)azo]-1-[di(2-hydroxyethyl)amino]-3-methylbenzol (HC Yellow No. 7), 2,6-Diamino-3-[(pyridin-3-yl)azo]-pyridin, 6-Hydroxy-5-[(4-sulfophenyl)azo]-2-naphthalinsulfonsäure-dinatriumsalz (CI15985; Food Yellow No. 3), 2,4-Dinitro-1-naphthol-7-sulfonsäure-dinatriumsalz (Cl10316; Acid Yellow No. 1; Food Yellow No. 1), 2-(Indan-1,3-dion-2-yl)chinolin-sulfonsäure (CI47005; Food Yellow No. 13; Acid Yellow No. 3), 5-Hydroxy-1-(4-sulfophenyl)-4-[(4-sulfophenyl)azo]pyrazol-3-carbonsäure-trinatriumsalz (Cl19140; Food Yellow No. 4; Acid Yellow No. 23), 9-(2-Carboxyphenyl)-6-hydroxy-3H-xanthen-3-on (CI45350; Acid Yellow No. 73), 5-[(2,4-Dinitrophenyl)amino]-2-phenylamino-benzolsulfonsäurenatriumsalz (CI10385; Acid Orange No. 3), 4-[(2,4-Dihydroxyphenyl)azo]-benzolsulfonsäure-mononatriumsalz (CI14270; Acid Orange No. 6), 4-[(2-Hydroxynaphth-1-yl)azo]-benzolsulfonsäure-natriumsalz (CI15510; Acid Orange No. 7), 4-[(2,4-Dihydroxy-3-[(2,4-dimethylphenyl)azo]-phenyl)azo]-benzolsulfonsäure-natriumsalz (CI20170; Acid Orange No. 24), 4-Hydroxy-3-[(4-sulfonaphth-1-yl)azo]-1-naphthalin-sulfonsäuredinatriumsalz (CI14720; Acid Red No. 14), 6-Hydroxy-5-[(4-sulfonaphth-1-yl)azo]-2,4-naphthalin-disulfonsäure-trinatriumsalz (CI16255; Ponceau 4R; Acid Red No. 18), 3-Hydroxy-4-[(4-sulfonaphth-1-yl)azo]-2,7-naphthalin-disulfonsäure-trinatriumsalz (CI16185; Acid Red No. 27), 8-Amino-1-hydroxy-2-(phenylazo)-3,6-naphthalin-disulfonsäuredinatriumsalz (CI17200; Acid Red No. 33), 5-(Acetylamino)-4-hydroxy-3-[(2-methylphenyl)azo]-2,7-naphthalin-disulfonsäure-dinatriumsalz (CI18065; Acid Red No. 35), 2-(3-Hydroxy-2,4,5,7-tetraiod-dibenzopyran-6-on-9-yl)-benzoesäure-dinatriumsalz (Cl45430; Acid Red No. 51), N-[6-(Diethylamino)-9-(2,4-disulfophenyl)-3H-xanthen-3-yliden]-N-ethylethanammonium-hydroxid, inneres Salz, Natriumsalz (CI45100; Acid Red No. 52), 8-[(4-(Phenylazo)phenyl)azo]-7-naphthol-1,3-disulfonsäuredinatriumsalz (CI27290; Acid Red No. 73), 2',4',5',7'-Tetrabrom-3',6'-dihydroxyspiro[isobenzofuran-1(3H),9'-[9H]xanthen]-3-on-dinatriumsalz (Cl45380; Acid Red No. 87), 2',4',5',7'-Tetrabrom-4,5,6,7-tetrachlor-3',6'-dihydroxyspiro[isobenzofuran-1(3H),9'[9H]xanthen]-3-on-dinatriumsalz (CI45410; Acid Red No. 92), 3',6'-Dihydroxy-4',5'-diiodospiro-[isobenzofuran-1(3H),9'(9H)-xanthen]-3-on-dinatriumsalz (CI45425; Acid Red No. 95), (2-Sulfophenyl)di[4-(ethyl((4-sulfophenyl)methyl)amino)phenyl]-carbenium-dinatriumsalz, betain (Cl42090; Acid Blue No. 9; FD&C Blue No. 1), 1,4-Bis[(2-sulfo-4-methylphenyl)amino]-9,10-anthrachinon-dinatriumsalz (CI 61570; Acid Green No. 25), Bis[4-(dimethylamino)phenyl]-(3,7-disulfo-2-hydroxynaphth-1-yl)carbenium-inneres Salz, mononatriumsalz (CI44090; Food Green No. 4; Acid Green No. 50), Bis[4-(diethylamino)phenyl](2,4-disulfophenyl)carbenium-inneres salz, Natriumsalz (2:1) (Cl42045; Food Blue No. 3; Acid Blue No. 1), Bis[4-(diethylamino)phenyl](5-hydroxy-2,4-disulfophenyl)carbenium-inneres salz, Calciumsalz (2:1) (CI42051; Acid Blue No. 3), 1-Amino-4-(cyclohexylamino)-9,10-anthrachinon-2-sulfonsäure-natriumsalz (CI62045; Acid Blue No. 62), 2-(1,3-Dihydro-3-oxo-5-sulfo-2H-indol-2-yliden)-2,3-dihydro-3-oxo-1H-indol-5-sulfonsäure-dinatriumsalz (CI73015; Acid Blue No. 74), 9-(2-Carboxyphenyl)-3-[(2-methylphenyl)amino]-6-[(2-methyl-4-sulfophenyl)amino]xanthylium-inneres Salz, mononatriumsalz (Cl45190; Acid Violet No. 9), 1-Hydroxy-4-[(4-methyl-2-sulfophenyl)amino]-9,10-anthrachinon-natriumsalz (Cl60730; D&C Violett No. 2; Acid Violet No. 43), Bis[3-nitro-4-[(4-phenylamino)-3-sulfo-phenylamino]-phenyl]-sulfon (Cl10410; Acid Brown No. 13), 5-Amino-4-hydroxy-6-[(4-nitrophenyl)azo]-3-(phenylazo)-2,7-naphthalin-disulfonsäure-dinatriumsalz (CI20470; Acid Black No. 1), 3-Hydroxy-4-[(2-hydroxynaphth-1-yl)azo]-7-nitro-1-naphthalin-sulfonsäure-chromkomplex (3:2) (CI15711; Acid Black No. 52), 3-[(2,4-Dimethyl-5-sulfophenyl)azo]-4-hydroxy-1-naphthalinsulfonsäure-dinatriumsalz (CI14700; Food Red No. 1; Ponceau SX; FD&C Red No. 4), 4-(Acetylamino)-5-hydroxy-6-[(7-sulfo-4-[(4-sulfophenyl)azo]naphth-1-yl)azo]-1,7-naphthalindisulfonsäuretetranatriumsalz (CI28440; Food Black No. 1), 3-Hydroxy-4-(3-methyl-5-oxo-1-phenyl-4,5-dihydro-1H-pyrazol-4-ylazo)-naphthalin-1-sulfonsäurenatriumsalz, Chrom-Komplex (Acid Red No. 195).

Die vorstehend genannten direktziehenden Nitrofarbstoffe, Azofarbstoffe, Anthrachinonfarbstoffe oder Triphenylmethanfarbstoffe können in einer Gesamtmenge von etwa 0,01 bis 4 Gewichtsprozent enthalten sein, wobei der Gesamtgehalt an nicht-oxidativen Farbstoffen vorzugsweise etwa 0,01 bis 8 Gewichtsprozent, insbesondere 0,1 bis 5 Gewichtsprozent, beträgt.

Die Zubereitungsform des erfindungsgemäßen Färbemittels auf der Basis der erfindungsgemäßen Verbindungen sowie gegebenenfalls , direktfärbenden Farbstoffen kann beispielsweise eine Lösung, insbesondere eine wäßrig-alkoholische Lösung sein. Bevorzugte Zubereitungsformen sind weiterhin Cremes, Gele oder Emulsionen. Ebenfalls ist es möglich, diese Mittel mit Hilfe eines Zerstäubers beziehungsweise anderer geeigneter Pumpvorrichtungen oder Sprühvorrichtungen oder im Gemisch mit üblichen unter Druck verflüssigten Treibmitteln als Aerosolspray oder Aerosolschaum aus einem Druckbehälter zu entnehmen.

Der pH-Wert dieses Färbemittels liegt im Bereich von 3 bis 12, insbesondere bei einem pH-Wert von 8 bis 11,5, wobei die Einstellung des gewünschten alkalischen pH-Wertes vorzugsweise mit Ammoniak erfolgt, jedoch auch mit organischen Aminen wie beispielsweise Monoethanolamin oder Triethanolamin vorgenommen werden kann, während für die Einstellung eines sauren pH-Wertes je nach gewünschten pH-Wert verdünnte organische oder anorganische Säuren, wie zum Beispiel Salzsäure, Schwefelsäure, Phosphorsäure, Ascorbinsäure oder Milchsäure, verwendet werden.

Die Anwendung dieses Färbemittels zu Haarfärbung erfolgt in üblicher Weise durch Aufbringen einer für die Färbung ausreichenden Menge des Mittels auf die Haare, mit denen es eine Zeit lang, vorzugsweise 15 bis 30 Minuten, in Kontakt bleibt. Anschließend wird das Haar mit Wasser, sodann gegebenenfalls noch mit einer wäßrigen Lösung einer schwachen organischen Säure gespült und abschließend getrocknet. Als schwache organische Säure können beispielsweise Essigsäure, Zitronensäure oder Weinsäure in Form einer verdünnten wäßrigen Lösung Verwendung finden.

Das vorstehend beschriebene Färbemittel ohne Oxidationsmittelzugabe kann weiterhin für kosmetische Mittel übliche natürliche oder synthetische Polymere beziehungsweise modifizierte Polymere natürlichen Ursprungs enthalten, wodurch gleichzeitig mit der Färbung eine Festigung der Haare erreicht wird. Solche Mittel werden im allgemeinen als Tönungsfestiger oder Farbfestiger bezeichnet.

Von den für diesen Zweck in der Kosmetik bekannten synthetischen Polymeren seien beispielsweise Polyvinylpyrrolidon, Polyvinylacetat, Polyvinylalkohol oder Polyacrylverbindungen wie Polyacrylsäure oder Polymethacrylsäure, basische Polymerisate von Estern der Polyacrylsäure, Polymethylacrylsäure und Aminoalkoholen beispielsweise deren Salze oder Quaternisierungsprodukte, Polyacrylnitril, Polyvinylacetate sowie Copolymerisate aus derartigen Verbindungen, wie zum Beispiel Polyvinylpyrrolidon-Vinylacetat, erwähnt; während als natürliche Polymere oder modifizierte natürliche Polymere beispielsweise Chitosan (entacetyliertes Chitin) oder Chitosanderivate, eingesetzt werden können.

Die vorgenannten Polymere können in dem erfindungsgemäßen Mittel in der für solche Mittel üblichen Mengen, insbesondere in einer Menge von etwa 1 bis 5 Gewichtsprozent, enthalten. Der pH-Wert des erfindungsgemäßen Tönungsfestigers oder Farbfestigers beträgt etwa 6 bis 9.

Die Anwendung des Haarfärbemittels mit zusätzlicher Festigung erfolgt in bekannter und üblicher Weise durch Befeuchten des Haares mit dem Festiger, Festlegen (Einlegen) des Haares zur Frisur und anschließende Trocknung.

Selbstverständlich kann das vorstehend beschriebene Haarfärbemittel ohne Oxidationsmittelzugabe gegebenenfalls weitere für Haarfärbemittel übliche Zusätze, wie zum Beispiel Pflegestoffe, Netzmittel, Verdicker, Weichmacher, Konservierungsstoffe und Parfümöle sowie auch andere, nachstehend für Oxidationshaarfärbemittel aufgeführte übliche Zusätze enthalten.

Zum Gegenstand der vorliegenden Erfindung gehören auch, wie bereits erwähnt, Haarfärbemittel, bei denen die Zugabe eines Oxidationsmittels erforderlich ist. Diese Färbemittel enthalten außer den erfindungsgemäßen Farbstoffen, sowie gegebenenfalls weiteren üblichen direkt auf das Haar aufziehenden Farbstoffen aus der Gruppe bestehend aus Nitrofarbstoffen, Azofarbstoffen, Anthrachinonfarbstoffen und Triphenylmethanfarbstoffen, zusätzlich bekannte Oxidationsfarbstoff-Vorstufen, die einer oxidativen Behandlung bedürfen (Entwicklersubstanzen und Kupplersubstanzen).

Beispiele für Entwickler sind 1,4-Diamino-benzol (p-Phenylendiamin), 1,4-Diamino-2-methyl-benzol (p-Toluylendiamin), 1,4-Diamino-2,6-dimethylbenzol, 1,4-Diamino-2,5-dimethyl-benzol, 1,4-Diamino-2,3-dimethylbenzol, 2-Chlor-1,4-diaminobenzol, 4-Phenylamino-anilin, 4-Dimethylamino-anilin, 4-Diethylamino-anilin, 4-[Di(2-hydroxyethyl)amino]-anilin, 4-[(2-Methoxyethyl)amino]-anilin, 4-[(3-Hydroxypropyl)amino]-anilin, 1,4-Diamino-2-(2-hydroxyethyl)-benzol, 1,4-Diamino-2-(1-methylethyl)benzol, 1,3-Bis[(4-aminophenyl)(2-hydroxyethyl)amino]-2-propanol, 1,8-Bis(2,5-diaminophenoxy)-3,6-dioxaoctan, 4-Amino-phenol, 4-Amino-3-methyl-phenol, 4-Methylamino-phenol, 4-Amino-2-(aminomethyl)-phenol, 4-Amino-2-[(2-hydroxyethyl)-amino]methyl-phenol, 4-Amino-2-(methoxymethyl)-phenol, 4-Amino-2-(2-hydroxyethyl)-phenol, 5-Aminosalicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamino-1-(1-methylethyl)-1H-pyrazol, 4,5-Diamino-1-[(4-methylphenyl)methyl]-1H-pyrazol, 1-[(4-Chlorphenyl)methyl]-4,5-diamino-1H-pyrazol, 4,5-Diamino-1-methyl-1 H-pyrazol, 2-Amino-phenol, 2-Amino-6-methyl-phenol, 2-Amino-5-methyl-phenol. Die genannten Entwicklersubstanzen können sowohl einzeln als auch im Gemisch miteinander verwendet werden.

Beispiele für Kuppler sind 5-((2-Hydroxyethyl)amino)-2-methoxyanilin, N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methylbenzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxypyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxyessigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlorphenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-2-methyl-phenol, 3-[(2-Hydroxyethyl)amino)-phenol, 3-[(2-Methoxyethyl)amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxybenzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxyphenol, 3,4-Methylendioxy-anilin, 5-[(2-Hydroxyethyl)amino]-1,3-benzodioxol, 6-Brom-1-hydroxy-3,4-methylendioxy-benzol, 3,4-Diaminobenzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol, 2,3-Indolindion. Die genannten Kupplersubstanzen können sowohl einzeln als auch im Gemisch miteinander verwendet werden.

Als besonders bevorzugte Verbindungen kommen vor allem 1,4-Diamino-2-methyl-benzol, 4-[Di(2-hydroxyethyl)amino]-anilin, 1,4-Diamino-2-(2-hydroxyethyl)-benzol, 4-Amino-3-methylphenol, 4-Amino-2-(aminomethyl)-phenol, 2,4,5,6-Tetraamino-pyrimidin, 4,5-Diamino-1-(2-hydroxy-ethyl)-1H-Pyrazol, 4-Chlorresorcin, 3-Aminophenol, 5-Hydroxy-1,3-benzodioxol, 5-Amino-1,3-benzodioxol, 5-((2'-Hydroxyethyl)-amino)-1,3-benzodioxol, 2-Amino-4-[(2-hydroxyethyl)amino]-anisol, 2,4-Diamino-1-fluor-5-methylbenzol, 5-Amino-2-methylphenol, 1,3-Di(2,4-diaminophenoxy)-propan, 3-Amino-2-chlor-6-methyl-phenol, 1-Naphthol, ,1,3-Dyhydroxy-benzol, 1,3-Dihydroxy-2-methyl-benzol, 2,4-Diamino-1-(2-hydroxyethoxy)-benzol, 5-((2-Hydroxyethyl)amino)-2-methoxyanilin und 5-((3-Hydroxypropyl)amino)-2-methylphenol in Betracht.

Weitere für die Haarfärbung bekannte und übliche Oxidationsfarbstoffe werden unter anderem in dem Buch von E. Sagarin, "Cosmetics, Science and Technology" (1957), Interscience Publishers Inc., New York, Seiten 503 ff, sowie in dem Buch von H. Janistyn, "Handbuch der Kosmetika und Riechstoffe, Band 3" (1973), Seite 388 bis 397, beschrieben.

Mit Kombinationen aus Oxidationsfarbstoffen und den erfindungsgemäßen Farbstoffen lassen sich sehr gut natürliche Blondtöne und Brauntöne, aber auch modische Nuancen herstellen.

Die erfindungsgemäßen Farbstoffe sind in dem Färbemittel mit Oxidationsmittel-Zugabe vorzugsweise in einer Menge von etwa 0,01 bis 4,0 Gewichtsprozent, insbesondere 0,02 bis 2,0 Gewichtsprozent, enthalten, wobei der Gesamtgehalt an nicht-oxidativen Farbstoffen vorzugsweise etwa 0,1 bis 5 Gewichtsprozent beträgt.

Die Gesamtkonzentration an Oxidationsfarbstoff-Vorstufen beträgt in dem erfindungsgemäßen Oxidationsfärbemittel etwa 0,1 und 10 Gewichtsprozent, wobei eine Menge von 0,2 bis 2 Gewichtsprozent bevorzugt ist.

Das Oxidationsfärbemittel kann sowohl sauer oder neutralals auch basisch (pH = 3 bis 12) eingestellt sein, wobei ein pH-Wert von 6 bis 12, insbesondere ein pH-Wert von etwa 8,0 bis 11,5, bevorzugt ist. Die Einstellung des gewünschten alkalischen pH-Wertes erfolgt hierbei in der Regel mit Ammoniak; es können aber auch andere organische Amine, zum Beispiel Monoethanolamin oder Triethanolamin, oder anorganische Basen wie Natriumhydroxid und Kaliumhydroxid verwendet werden, während die Einstellung eines sauren pH-Wertes mit organischen Säuren, wie zum Beispiel Citronensäure, Glycolsäure, Milchsäure, Äpfelsäure, Ascorbinsäure oder Weinsäure, erfolgt.

Als Oxidationsmittel zur Entwicklung der Färbung kommen in erster Linie Wasserstoffperoxid und dessen Additionsverbindungen in Betracht. Die Zubereitungsform des erfindungsgemäßen Oxidationsfärbemittels kann die gleiche wie bei dem erfindungsgemäßen nicht-oxidativen Färbemittel sein, wobei das Oxidationsfärbemittel vorzugsweise in Form einer Creme oder eines Gels vorliegt.

Übliche Zusätze in Lösungen, Cremes, Emulsionen oder Gelen sind zum Beispiel Lösungsmittel wie Wasser, niedere aliphatische einwertige oder mehrwertige Alkohole, deren Ester und Ether, beispielsweise Alkanole, insbesondere mit 1 bis 4 C-Atomen, beispielsweise Ethanol, Propanol oder Isopropanol, Butanol, Isobutanol, zweiwertige und dreiwertige Alkohole, insbesondere solche mit 2 bis 6 C-Atomen, beispielsweise Ethylenglycol, Propylenglycol, 1,3-Propandiol, 1,4-Butandiol, 1,5-Pentandiol, 1,6-Hexandiol, 1,2,6-Hexantriol, Glycerin, Diethylenglycol, Dipropylenglycol, Polyalkylenglycole, wie Triethylenglycol, Polyethylenglycol, Tripropylenglycol, Polypropylenglycol, niedere Alkylether von mehrwertigen Alkoholen, wie Ethylenglycolmonomethylether oder Ethylenglycolmonoethylether oder Ethylenglycolmonopropylether oder Ethylenglycolmonobutylether, Diethylenglycolmonomethylether oder Diethylenglycolmonoethylether, Triethylenglycolmonomethylether oder Triethylenglycolmonoethylether, Ketone und Ketoalkohole, insbesondere solche mit 3 bis 7 C-Atomen, wie zum Beispiel Aceton, Methylethylketon, Diethylketon, Methylisobutylketon, Methylphenylketon, Cyclopentanon, Cyclohexanon und Diacetonalkohol, Ether, wie z.B. Dibutylether, Tetrahydrofuran, Dioxan, Diisopropylether, Ester wie zum Beispiel Ethylformiat, Methylformiat, Methylacetat, Ethylacetat, Propylenacetat, Butylacetat, Phenylacetat, Ethylenglycolmonoethyletheracetat und Essigsäurehydroxyethylester, Amide wie zum Beispiel Dimethylformamid und Dimethylacetamid, N-Methylpyrrolidon, sowie Harnstoff, Tetramethylhamstoff und Thiodiglycol.

Weiterhin können in dem erfindungsgemäßen Färbemittel Netzmittel oder Emulgatoren aus den Klassen der anionischen, kationischen, amphoteren, nichtionogenen oder zwitterionischen oberflächenaktiven Substanzen wie Fettalkoholsulfate, Alkansulfonate, Alkylbenzolsulfonate, Alkyltrimethylammoniumsalze, Alkylbetaine, α-Olefinsulfonate, oxethylierte Fettalkohole, oxethylierte Nonylphenole, Fettsäurealkanolamine, oxethylierte Fettsäureester, Fettalkoholpolyglycolethersulfate, Alkylpolyglucoside, Verdickungsmittel wie höhere Fettalkohole, Stärke, Alginate, Bentonite, Cellulosederivate, Vaseline, Paraffinöl und Fettsäuren, wasserlösliche polymere Verdickungsmittel wie natürliche Gummiarten, Guargummi, Xanthangummi, Johannisbrotkernmehl, Pektin, Dextran, Agar-Agar, Amylose, Amylopektin, Dextrine, Tone oder vollsynthetische Hydrokolloide wie Polyvinylalkohol, außerdem Pflegestoffe wie Lanolinderivate, Cholesterin, Pantothensäure, wasserlösliche kationische Polymere, Proteinderivate, Provitamine, Vitamine, Pflanzenextrakte, Zucker und Betain, Hilfsstoffe wie Feuchthaltemittel, Elektrolyte, Antioxidantien, Fettamide, Sequestrierungsmittel, filmbildende Agentien und Konservierungsmittel, enthalten sein. Neben Wasser kann auch auch ein wasserlösliches organisches Lösungsmittel oder ein Gemisch derartiger Lösungsmittel sowie ein Wasser/Lösungsmittel-Gemisch verwendet werden.

Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Menge von etwa 0,1 bis 5 Gewichtsprozent.

Für die Anwendung zur oxidativen Färbung von Haaren vermischt man das vorstehend beschriebene Färbemittel unmittelbar vor dem Gebrauch mit einem Oxidationsmittel und trägt eine für die Haarfärbebehandlung ausreichende Menge, je nach Haarfülle, im allgemeinen etwa 60 bis 200 g, dieses Gemisches auf das Haar auf. Als Oxidationsmittel zur Entwicklung der Haarfärbung kommen hauptsächlich Wasserstoffperoxid oder dessen Additionsverbindungen an Harnstoff, Melanin oder Natriumborat in Form der 3- bis 12%igen, vorzugsweise 6%igen wäßrigen Lösungen, in Betracht.

Wird eine 6%ige Wasserstoffperoxidlösung als Oxidationsmittel verwendet, so beträgt das Gewichtsverhältnis zwischen Färbemittel und Oxidationsmittel 5:1 bis 1:2, vorzugsweise jedoch 1:1. Größere Mengen an Oxidationsmittel werden vor allem bei höheren Farbstoffkonzentrationen im Färbemittel oder wenn gleichzeitig eine stärkere Bleichung des Haares beabsichtigt ist, verwendet.

Man läßt die Färbelösung bei 20 bis 50 °C etwa 10 bis 45 Minuten lang, vorzugsweise 40 °C bei 40 Minuten lang, auf das Haar einwirken, spült sodann das Haar mit Wasser aus und trocknet es. Gegebenenfalls wird im Anschluß an diese Spülung mit einem Shampoo gewaschen und nachgespült. Anschließend wird das Haar getrocknet.

Das erfindungsgemäße Färbemittel führt - insbesondere bei Haarfärbungen - zu Färbungen mit ausgezeichneten Echtheitseigenschaften, insbesondere was die Lichtechtheit, Waschechtheit und Schweißechtheit anbetrifft. Bemerkenswert ist die große Farbintensität und die Farbreinheit der erzielbaren Färbungen auch nach dreimonatiger Lagerung. Schließlich ist mit Hilfe des beschriebenen Färbemittels auch eine Anfärbung von ergrautem und chemisch nicht vorgeschädigtem Haar problemlos und mit guter Deckkraft möglich. Die dabei erhaltenen Färbungen sind, unabhängig von der unterschiedlichen Struktur des Haares, gleichmäßig und sehr gut reproduzierbar. Hierbei werden die Haarfärbemittel je nach Zusammensetzung entweder in Verbindung mit oder auch ohne Oxidationsmittel angewandt.

Die erfindungsgemäßen Färbemittel auf der Basis von Verbindungen der Formel (I) sind besonders gut zur Färbung von Keratinfasem, wie zum Beispiel Wolle, Pelzen oder Haaren, insbesondere menschlichen Haaren, geeignet; es ist jedoch auch ohne weiteres möglich, mit diesen Färbemitteln andere Naturfasern, wie zum Beispiel Baumwolle, Jute, Sisal, Leinen oder Seide, modifizierte Naturfasern, wie zum Beispiel Regeneratcellulose, Nitrocellulose, Alkylcellulose, Hydroxyalkylcellulose oder Acetylcellulose, oder synthetische Fasern, wie zum Beispiel Polyamidfasern, Polyurethanfasern und Polyesterfasern, zu färben.

Die nachfolgenden Beispiele sollen den Erfindungsgegenstand näher erläutern, ohne diesen hierauf zu beschränken.

### Beispiele

### Synthesebeispiele

### Beispiel 1: Synthese von N-(2-Methoxyethyl)-4-nitro-phenylamin

28,2 g (0,2 mol) 1-Fluor-4-nitrobenzol werden mit 87,1 g (1,16 mol) 1-Amino-2-methoxyethan unter Rückfluß 5 Stunden erhitzt. Nach Beendigung der Reaktion (DC-Kontrolle; Essigsäureethylester) wird das Reaktionsprodukt auf 500 g Eis/Wasser gegossen. Es fällt ein gelber Farbstoff aus. Umkristallisation aus Ethanol ergibt feine gelbe Nadeln mit einem Schmelzpunkt von 85-87 °C.
Ausbeute: 97% der Theorie.
¹H-NMR (60 MHz. DMSO-d₆): 1,13 (t, CH₃, 3H); 3,34 (s, CH, 1 H); 3,93 (q, CH₂, 2H); 7,55 (d, H-(C6), 1 H); 7,95 (dxd, H-C(5), 1 H); 8,27 (d, H-C(3), 1 H)
IR-Spektrum (Film): 3110 w, 2987 m, 2250 w, 1749 vs, 1610 s, 1542 s, 1468 s, 1350 s, 1261 s, 1216 s, 1026 s
MS (m/z, rel. Intensität): 196 (50, M⁺), 180 (14, CH₄⁺), 151 (63, -NO₂), 135 (9), 119 (6), 105 (77), 104 (13)
DC (Hexan/ Ethylacetat 3:1): *R*_{f} = 0,38.

| Elementaranalyse: C₉H₁₂O₃N₂ (MG = 196,21): | | | |
|---|---|---|---|
| | C | H | N |
| berechnet | 55,08 | 6,17 | 14,28 |
| gefunden | 55,21 | 6,14 | 14,33 |

### Beispiel 2: Synthese von N-(2-Methoxyethyl)-2-methyl-4-nitro-phenylamin

26 g (0,17 mol) 1-Fluor-2-methyl-4-nitrobenzol werden mit 73 g (1 mol) 1-Amino-2-methoxyethan unter Rückfluß 5 Stunden lang erhitzt. Danach wird das Reaktionsprodukt auf 500 g Eis/Wasser gegossen. Der erhaltene Feststoff wird abfiltriert und aus Ethanol umkristallisiert. Es werden kleine gelbe Prismen mit einem Schmelzpunkt von 97-100 °C erhalten.
Ausbeute: 74% der Theorie
¹H-NMR (60 MHz. DMSO-d₆): 1,13 (t, CH₃, 3H); 3,34 (s, CH, 1H); 3,93 (q, CH₂, 2H); 7,55 (d, H-(C6), 1H); 7,95 (dxd, H-C(5), 1 H); 8,27 (d, H-C(3), 1 H)
IR-Spektrum (Film): 3110 w, 2987 m, 2250 w, 1749 vs, 1610 s, 1542 s, 1468 s, 1350 s, 1261 s, 1216 s, 1026 s
MS (m/z. rel. Intensität): 210 (30, M⁺), 165 (100, -NO₂), 119 (45, C₇H₇), 118 (10), 107 (5), 104 (8), 89 (9)

| Elementaranalyse: C₁₀H₁₄O₂N₃ (210,23): | | | |
|---|---|---|---|
| | C | H | N |
| berechnet | 57,12 | 6,72 | 13,33 |
| gefunden | 57,16 | 6,71 | 13,40 |

### Beispiel 3: Synthese von N-(3-Methoxypropyl)-4-nitro-phenylamin

10 g (70,8 mmol) 1-Fluor-4-nitrobenzol werden mit 36,5 g (410 mmol) 3-Methoxy-propylamin unter Rückfluß 5 Stunden lang erhitzt. Danach wird das Reaktionsprodukt auf 400 g Eis/Wasser gegossen und der Farbstoff abfiltriert. Es wird ein gelber Farbstoff mit einem Schmelzpunkt von 57-59 °C erhalten.
Ausbeute: 98% der Theorie

### Beispiel 4: Synthese von N-(3-Methoxypropyl)-2-methyl-4-nitro-phenylamin

10 g (0,07 mol) 1-Fluor-2-methyl-4-nitrobenzol werden mit 36,5 g (0,4 mol) 1-Amino-2-methoxypropan unter Rückfluß 5 Stunden lang erhitzt. Danach wird das Reaktionsprodukt auf 350 g Eis/Wasser gegossen und der Farbstoff abfiltriert. Nach Umkristallisation aus Ethanol wird ein gelbes kristallines Pulver mit einem Schmelzpunkt von 26-28 °C erhalten.
Ausbeute: 60% der Theorie.

### Beispiele für Färbemittel

### Beispiel 5: Haarfärbemittel mit N-(2-Methoxyethyl)-4-nitro-phenylamin

| | |
|---|---|
| 0,49 g | N-(2-Methoxyethyl)-4-nitro-phenylamin |
| 10,00 g | Isopropanol |
| 10,00 g | Laurylalkohol-diglycolethersulfat-Natriumsalz (28%ige wässerige Lösung) |
| ad 100,00 g | Wasser |

Die vorstehende Farbträgermasse wird unmittelbar vor Gebrauch zum gebrauchsfertigen Haarfärbemittel vermischt. Die Einstellung des pH-Wertes erfolgt einmal mit Milchsäure (sauer: pH=5) und einmal mit verdünnter Ammoniaklösung (alkalisch: pH=8). Gebleichte Haare werden sodann 40 Minuten bei einer Temperatur von 40° C mit den beiden Haarfärbemitteln ( einmal sauer und einmal basisch eingestellt) behandelt. Anschließend werden die Haare mit Wasser gespült und getrocknet.
Es resultiert sowohl nach saurer als auch basischer Behandlung eine intensive zitronengelbe Färbung. Die erhaltene Färbung übersteht bis zu fünf Haarwäschen oder eine dreiwöchige Exposition unter Sonnenlicht ohne merklichen Intensitätsverlust.

### Beispiel 6: Haarfärbemittel mit N-(2-Methoxyethyl)-2-methyl-4-nitrophenylamin

| | |
|---|---|
| 0,525 g | N-(2-Methoxyethyl)-2-methyl-4-nitro-phenylamin |
| 10,000 g | Isopropanol |
| 10,000 g | Laurylalkohol-diglycolethersulfat-Natriumsalz (28%ige wässerige Lösung) |
| ad 100,000 g | Wasser |

Das gebrauchsfertige Haarfärbemittel wird unmittelbar vor Gebrauch durch Vermischen der übrigen Bestandteile mit Wasser hergestellt. Die Einstellung des pH-Wertes erfolgt einmal mit Milchsäure (sauer: pH=5) und einmal mit verdünnter Ammoniaklösung (alkalisch: pH=8). Gebleichte Haare werden sodann 40 Minuten bei einer Temperatur von 40° C mit den beiden Haarfärbemitteln ( einmal sauer und einmal basisch eingestellt) behandelt. Anschließend werden die Haare mit Wasser gespült und getrocknet.
Es resultiert sowohl nach saurer als auch basischer Behandlung eine intensive maisgelbe Färbung. Die erhaltene Färbung übersteht bis zu fünf Haarwäschen oder eine dreiwöchige Exposition unter Sonnenlicht ohne merklichen Intensitätsverlust.

### Beispiel 7: Haarfärbemittel mit N-(3-Methoxy-propyl)-4-nitro-phenylamin

| | |
|---|---|
| 0,525 g | N-(3-Methoxy-propyl)-4-nitro-phenylamin |
| 10,000 g | Isopropanol |
| 10,000 g | Laurylalkohol-diglycolethersulfat-Natriumsalz (28%ige wässerige Lösung) |
| ad 100,000 g | Wasser |

Das gebrauchsfertige Haarfärbemittel wird unmittelbar vor Gebrauch durch Vermischen der übrigen Bestandteile mit Wasser hergestellt. Die Einstellung des pH-Wertes erfolgt einmal mit Milchsäure (sauer: pH=5) und einmal mit verdünnter Ammoniaklösung (alkalisch: pH=8). Gebleichte Haare werden sodann 40 Minuten bei einer Temperatur von 40° C mit den beiden Haarfärbemitteln ( einmal sauer und einmal basisch eingestellt) behandelt. Anschließend werden die Haare mit Wasser gespült und getrocknet.
Es resultiert sowohl nach saurer als auch basischer Behandlung eine intensive gelbe Färbung. Die erhaltene Färbung übersteht bis zu fünf Haarwäschen oder eine dreiwöchige Exposition unter Sonnenlicht ohne merklichen Intensitätsverlust.

### Beispiel 8: Haarfärbemittel mit N-(3-Methoxypropyl)-2-methyl-4-nitro-phenylamin

| | |
|---|---|
| | |
| 0,56 g | N-(3-Methoxypropyl)-2-methyl-4-nitro-phenylamin |
| 10,00 g | Isopropanol |
| 10,00 g | Laurylalkohol-diglycolethersulfat-Natriumsalz (28%ige wässerige Lösung) |
| ad 100,00 g | Wasser |

Das gebrauchsfertige Haarfärbemittel wird unmittelbar vor Gebrauch durch Vermischen der übrigen Bestandteile mit Wasser hergestellt. Die Einstellung des pH-Wertes erfolgt einmal mit Milchsäure (sauer: pH=5) und einmal mit verdünnter Ammoniaklösung (alkalisch: pH=8). Gebleichte Haare werden sodann 40 Minuten bei einer Temperatur von 40° C mit den beiden Haarfärbemitteln ( einmal sauer und einmal basisch eingestellt) behandelt. Anschließend werden die Haare mit Wasser gespült und getrocknet.
Es resultiert sowohl nach saurer als auch basischer Behandlung eine intensive gelbe Färbung. Die erhaltene Färbung übersteht bis zu fünf Haarwäschen oder eine dreiwöchige Exposition unter Sonnenlicht ohne merklichen Intensitätsverlust.

### Beispiel 9: Oxidationshaarfärbemittel in Cremeform

| | |
|---|---|
| 0,2 g | N-(2-Methoxyethyl)-2-methyl-4-nitro-phenylamin |
| 15,0 g | Cetylalkohol |
| 3,5 g | Laurylalkohol-diglykolethersulfat-Natriumsalz (28%ige wäßrige Lösung) |
| 0,4 g | Natriumsulfit |
| | |
| 0,1 g | 3-Aminophenol |
| 0,2 g | 1,3-Dihydroxy-2-methylbenzol |
| 0,4 g | 1,4-Diamino-2-methyl-benzol |
| 40,0 g | Ammoniak (25%ige wäßrige Lösung) |
| ad 100,0 g | Wasser, vollentsalzt |

50 g des vorstehenden Haarfärbemittels werden unmittelbar vor der Anwendung mit 50 g Wasserstoffperoxidlösung (6%ige wäßrige Lösung) vermischt und auf gebleichtes Haar aufgetragen. Nach einer Einwirkungszeit von 30 Minuten bei wird das Haar mit Wasser gespült, schamponiert und getrocknet.
Das Haar erhält eine braune Färbung.

### Beispiel 10: Oxidationshaarfärbemittel in Cremeform

| | |
|---|---|
| 0,03 g | N-(2-Methoxyethyl)-2-methyl-4-nitro-phenylamin |
| 23,00 g | Cetylalkohol |
| 10,00 g | Laurylalkohol-diglykolethersulfat-Natriumsalz (28%ige wäßrige Lösung) |
| 0,30 g | Ascorbinsäure |
| 0,40 g | Natriumsulfit |
| 0,03 g | 3-Aminophenol |
| 0,29 g | 1,3-Dihydroxybenzol |
| 0,03 g | 1,3-Dihydroxy-2-methylbenzol |
| 0,60 g | 1,4-Diamino-2-methyl-benzol |
| 0.01 g | Natriumhydroxid |
| 0,40 g | Isopropylalkohol |
| 7,60 g | Ammoniak (25%ige wäßrige Lösung) |
| ad 100,00 g | Wasser, vollentsalzt |

50 g des vorstehenden Haarfärbemittels werden unmittelbar vor der Anwendung mit 50 g Wasserstoffperoxidlösung (6%ige wäßrige Lösung) vermischt und auf gebleichtes Haar aufgetragen. Nach einer Einwirkungszeit von 30 Minuten bei wird das Haar mit Wasser gespült, schamponiert und getrocknet.
Das so behandelte Haar erhält eine nätürliche hellbraune Färbung

### Beispiel 11: Oxidationshaarfärbemittel in Cremeform

| | |
|---|---|
| 0,03 g | N-(2-Methoxyethyl)-2-methyl-4-nitro-phenylamin |
| 23,00 g | Cetylalkohol |
| 10,00 g | Laurylalkohol-diglykolethersulfat-Natriumsalz (28%ige wäßrige Lösung) |
| 0,30 g | Ascorbinsäure |
| 0,40 g | Natriumsulfit |
| 0,02 g | 4-Amino-3-methylphenol |
| 0,03 g | 1,4-Diamino-2-(hydroxyethyl)benzol-sulfat (1:1) |
| 0,01 g | 1,3-Di(2,4-diaminophenoxy)-propan tetrahydrochlorid |
| 0,01 g | 5-Amino-2-methylphenol |
| 0,02 g | 5-((2-Hydroxyethyl)amino)-2-methoxyanilin-sulfat (1:1)) |
| 0,01 g | 1,3-Diamino-4-(2-hydroxyethoxy)-benzol |
| 0,01 g | 4-Amino-2-(aminomethyl)phenol-dihydrochlorid |
| 0,02 g | 5-Amino-2-methylphenol |
| 0,03 g | 3-Aminophenol |
| 0,29 g | 1,3-Dihydroxybenzol |
| 0.03 g | 1,3-Dihydroxy-2-methylbenzol |
| 0,60 g | 1,4-Diamino-2-methyl-benzol |
| 0,01 g | Natriumhydroxid |
| 0,40 g | Isopropylalkohol |
| 7,60 g | Ammoniak (25%ige wäßrige Lösung) |
| ad 100,00 g | Wasser, vollentsalzt |

50 g des vorstehenden Haarfärbemittels werden unmittelbar vor der Anwendung mit 50 g Wasserstoffperoxidlösung (6%ige wäßrige Lösung) vermischt und auf gebleichtes Haar aufgetragen. Nach einer Einwirkungszeit von 30 Minuten bei wird das Haar mit Wasser gespült, schamponiert und getrocknet.
Es wird eine braune Färbung erhalten.

### Beispiel 12: Oxidationshaarfärbemittel in Cremeform

| | |
|---|---|
| 0,03 g | N-(2-Methoxyethyl)-4-nitro-phenylamin |
| 23,00 g | Cetylalkohol |
| 10,00 g | Laurylalkohol-diglykolethersulfat-Natriumsalz (28%ige wäßrige Lösung) |
| 0,30 g | Ascorbinsäure |
| 0,40 g | Natriumsulfit |
| 0,03 g | 1,4-Diamino-2-(hydroxyethyl)benzol-sulfat (1:1) |
| 0,01 g | 1,3-Di(2,4-diaminophenoxy)-propan tetrahydrochlorid |
| 0,02 g | 5-((2-Hydroxyethyl)amino)-2-methoxyanilin-sulfat (1:1)) |
| 0,01 g | 1,3-Diamino-4-(2-hydroxyethoxy)-benzol |
| 0,01 g | 4-Amino-2-(aminomethyl)phenol-dihydrochlorid |
| 0,01 g | 2,4-Diamino-1,5-di(2-hydroxyethoxy)-benzol |
| 0,01 g | 4-Amino-2-(((2-hydroxyethyl)-amino)-methyl)-phenol |
| 0,01 g | 2,4-Diamino-1-fluor-5-methyl-benzolsulfat-hydrat |
| 0,03 g | 1,3-Dihydroxy-2-methylbenzol |
| 0,01 g | Natriumhydroxid |
| 0,40 g | Isopropylalkohol |
| 7,60 g | Ammoniak (25%ige wäßrige Lösung) |
| ad 100,00 g | Wasser, vollentsalzt |

50 g des vorstehenden Haarfärbemittels werden unmittelbar vor der Anwendung mit 50 g Wasserstoffperoxidlösung (6%ige wäßrige Lösung) vermischt und auf gebleichtes Haar aufgetragen. Nach einer Einwirkungszeit von 30 Minuten bei wird das Haar mit Wasser gespült, schamponiert und getrocknet.
Das Haar wird in einem intensiven Braunton gefärbt.

### Beispiel 13: Haarfärbemittel mit Direktfarbstoffen

| | |
|---|---|
| 0,525 g | N-(2-Methoxyethyl)-2-methyl-4-nitro-phenylamin |
| 0,380 g | 4-(Di-(2-hydroxyethyl)-amino)-N-(2-hydroxyethyl)-2-nitroanilin |
| 0,358 g | 4-(Ethyl-(2-hydroxyethyl)-amino)-N-(2-hydroxyethyl)-2-nitroanilin |
| 0,0740 g | 4-(Di(2-hydroxyethyl)amino)-2-nitroanilin-hydrochlorid |
| 10,000 g | Isopropanol |
| 10,000 g | Laurylalkohol-diglycolethersulfat-Natriumsalz (28%ige wässerige Lösung) |
| ad 100,000 g | Wasser |

Die vorstehende Farbträgermasse wird unmittelbar vor Gebrauch zum gebrauchsfertigen Haarfärbemittel vermischt. Die Einstellung des pH-Wertes erfolgt einmal mit Milchsäure (sauer: pH=5) und einmal mit verdünnter Ammoniaklösung (alkalisch: pH=8). Gebleichte Haare werden sodann 40 Minuten bei einer Temperatur von 40° C mit dem Haarfärbemittel (einmal sauer und einmal basisch eingestellt) behandelt. Anschließend werden die Haare mit Wasser gespült und getrocknet.
Das so behandelte Haar erhält eine dunkelbraune Färbung.

### Beispiel 14: Färbemittel mit Direktfarbstoffen

| | |
|---|---|
| 0,490 g | N-(2-Methoxyethyl)-4-nitro-phenylamin |
| 0,709 g | 4-(Ethyl-(2-hydroxyethyl)-amino)-N-(2-hydroxyethyl)-2-nitroanilin |
| 0,064 g | 5-Chlor-4-((2,3-dihydroxypropyl)-amino)-2-nitroanilin |
| 0,088 g | 1-((4-aminophenyl)-azo)-2-hydroxy-7- (trimethyl-ammonium)-naphthalin |
| 10,000 g | Isopropanol |
| 10,000 g | Laurylalkohol-diglycolethersulfat-natriumsalz (28%ige wässerige Lösung) |
| ad 100,00 g | Wasser |

Die Einstellung des pH-Wertes erfolgt a) mit Milchsäure oder b) mit verdünnter Ammoniaklösung. Gebleichte Haare bzw. Baumwolle oder Wolle werden mit dem vorstehend beschriebenen Färbemittel 40 Minuten bei einer Temperatur von 40° C mit dem Haarfärbemittel (einmal sauer: pH=5 und einmal basisch: pH=8 eingestellt) behandelt. Anschließend werden die Fasern mit Wasser gespült und getrocknet.
Die so behandelten Fasern erhalten jeweils eine intensive dunkelbraune Färbung.

### Vergleichsversuche:

### a) Überprüfung des Auswaschverhaltens

Die Farbmeßwerte von 5 gefärbten Haarsträhnen werden bestimmt. Anschließend werden die Haarsträhnen jeweils 5mal hintereinander mit einem neutralen Shampoo 1 Minute lang shampooniert, sodann mit Wasser gespült und getrocknet. Im Anschluß an diese Behandlung werden die Farbmeßwerte der einzelnen Strähnen erneut bestimmt und die Differenz der Farbmeßwerte vor und nach der Behandlung ermittelt. Der Mittelwert der Ergebnisse der 5 Haarsträhnen ist in Tabelle 1 aufgelistet.

### b) Überprüfung der Schweißbeständigkeit

Die Farbmeßwerte von 5 gefärbten Haarsträhnen werden bestimmt. Anschließend werden die Haarsträhnen jeweils in die nachfolgend aufgeführte Lösung von Syntheseschweiß aus

| | |
|---|---|
| 10,00 g | Natriumchlorid |
| 1,00 g | di-Kaliumphosphat |
| 0,25 g | DL-Histidin |
| ad pH 3,2 | Milchsäure |
| ad 100,00 g | Wasser |

eingetaucht und zweimal für jeweils 24 Stunden bei 37°C in dieser Lösung belassen.

Sodann werden die Haarsträhnen mit Wasser ausgespült und getrocknet. Im Anschluß an diese Behandlung werden die Farbmeßwerte der einzelnen Strähnen erneut bestimmt und die Differenz der Farbmeßwerte vor und nach der Behandlung ermittelt.
Der Mittelwert der Ergebnisse der 5 Haarsträhnen ist in Tabelle 1 aufgelistet.

### c) Überprüfung der Lichtechtheit

Die Farbmeßwerte von 5 gefärbten Haarsträhnen werden bestimmt. Anschließend werden die gefärbten Haarsträhnen mit einem Schnellbelichtungsgerät der Marke "Atlas Suntest CPS+", mit einer Xenon Bogenentladungslampe 1,5 kW bestrahlt. Die Lichtechtheit wird bewertet, indem man die Änderung der Farbmeßwerte mit der der Typfärbungen des Lichtechtheitsmaßstabes vergleicht. Im Anschluß an diese Behandlung werden die Farbmeßwerte der einzelnen Strähnen erneut bestimmt und die Differenz der Farbmeßwerte vor und nach der Behandlung ermittelt. Der Mittelwert der Ergebnisse der 5 Haarsträhnen ist in Tabelle 1 aufgelistet.

Die in der nachfolgenden Tabelle 1 angegebenen L*a*b*-Farbmeßwerte wurden mit einem Farbmeßgerät der Firma Minolta, Typ Chromameter II, ermittelt. Die Gesamtfarbabstände (ΔE-Farbmeßwerte) sind gemäß dem CIE -Farbsystem L*a*b* angegeben. Hierbei steht der L-Wert für die Helligkeit (je geringer der L-Wert desto dunkler ist die Probe und je höher der L-Wert desto heller ist die Probe), während der a-Wert ein Maß für den Rotanteil bzw. Grünanteil ist (je größer bzw. positiver der a-Wert ist umso größer ist der Rotanteil und je kleiner bzw. negativer der a-Wert ist desto größer ist der Grünanteil). Der b-Wert ist ein Maß für den Blauanteil bzw. Gelbanteil (je größer bzw. positiver der b-Wert ist umso größer ist der Gelbanteil und je kleiner bzw. negativer der b-Wert ist desto größer ist der Blauanteil). Der ΔE-Wert ist ein Maß für die Farbänderung, wobei der Intensitätsverlust der Färbung umso größer ist je größer der ΔE-Wert ist.

**Tabelle 1:**

| **Färbemittel gemäß** | **Auswaschverhalten ΔE-Werte**^{***)**} | **Schweissbeständigkeit ΔE-Werte**^{***)**} | **Lichtechtheit ΔE-Werte**^{***)**} |
|---|---|---|---|
| **Beispiel 1** | 26,7 | 35,0 | 42,7 |
| **Beispiel 2** | 16,2 | 31,2 | 38,8 |
| **Vergleichsversuch:** (Färbemittel gemäß Beispiel 1, in dem das erfindungsgemäße N-(2-Methoxyethyl)-4-nitro-phenylamin durch einen üblichen direktziehenden Farbstoff [N-(2-Hydroxyethyl)-4-nitro-phenylamin] ersetzt wurde) | 30,9 | 46,5 | 42,1 |

| | | | |
|---|---|---|---|
| ^{*)} gemittelt über 5 Haarsträhnchen | | | |

Wie sich aus den Meßwerten ersehen läßt, besitzen die erfindungsgemäßen Haarfärbemittel (Beispiel 1 + 2) eine gegenüber einem üblichen Haarfärbemittel höhere Stabilität der Färbungen gegenüber Auswaschen sowie dem Einfluß von Schweiß und Lichtbestrahlung.

Alle in der vorliegenden Anmeldung genannten Prozentangaben stellen soweit nicht anders angegeben Gewichtsprozente dar.

## Patentansprüche

1. Mittel zum Färben von Fasern, **dadurch gekennzeichnet, daß** es mindestens eine Verbindung aus der Gruppe bestehend aus N-(2-Methoxyethyl)-4-nitro-phenylamin, N-(2-Methoxyethyl)-2-methyl-4-nitro-phenylamin, N-(3-Methoxypropyl)-4-nitro-phenylamin und N-(3-Methoxypropyl)-2-methyl-4-nitro-phenylamin enthält.

2. Mittel nach Anspruch 1, **dadurch gekennzeichnet, daß** die Verbindung der Formel (I) in einer Menge von 0,01 und 10 Gewichtsprozent enthalten ist.

3. Mittel nach Anspruch 1 oder 2, **dadurch gekennzeichnet, daß** es einen pH-Wert von 3 bis 12 aufweist.

4. Mittel nach einem der Ansprüche 1 bis 3 dadurch gekennzeichet, daß es zusätzlich 0,1 bis 10 Gewichtsprozent Oxidationsfarbstoff-Vorstufen, welche einer oxidativen Behandlung bedürfen (Entwicklersubstanzen und Kupplersubstanzen), enthält.

5. Mittel nach einem der Ansprüche 1 bis 4, **dadurch gekennzeichnet, daß** es zusätzlich 0,01 bis 4 Gewichtsprozent eines direktziehenden Farbstoffes aus der Gruppe bestehend aus Nitrofarbstoffen, Azofarbstoffen, Anthrachinonfarbstoffen und Triphenylmethanfarbstoffen enthält.

6. Mittel nach einem der Ansprüche 1 bis 3 oder 5, **dadurch gekennzeichnet, daß** es zusätzlich 1 bis 5 Gewichtsprozent eines natürlichen oder synthetischen Polymers beziehungsweise eines modifizierten Polymers natürlichen Ursprungs enthält und einen pH-Wert von 6 bis 9 aufweist.

7. Verfahren zum Färben von Haaren, **dadurch gekennzeichnet, daß** eine für die Färbung ausreichenden Menge eines Mittels nach einem der Ansprüche 1 bis 3, 5 oder 6 auf die Haare aufgetragen wird, nach einer Einwirkungszeit von 15 bis 30 Minuten das Haar mit Wasser ausgespült und getrocknet wird.

8. Verfahren zum Färben von Haaren, **dadurch gekennzeichnet, daß** eine für die Färbung ausreichenden Menge eines Mittels nach einem der Ansprüche 1 bis 4 unmittelbar vor dem Gebrauch mit einem Oxidationsmittel vermischt wird, eine für die Haarfärbebehandlung ausreichende Menge des erhaltenen gebrauchsfertigen Oxidationsfärbemittels auf das Haar aufgetragen wird und nach einer Einwirkungszeit von 10 bis 45 Minuten bei 20 bis 50 °C das Haar mit Wasser ausgespült und getrocknet wird.

9. Verwendung eines Mittels nach einem der Ansprüche 1 bis 6 zur Färbung von Haaren.

## Claims

1. Composition for the colouring of fibres, **characterized in that** it comprises at least one compound from the group consisting of N-(2-methoxyethyl)-4-nitrophenylamine, N-(2-methoxyethyl)-2-methyl-4-nitrophenylamine, N-(3-methoxypropyl)-4-nitrophenylamine and N-(3-methoxypropyl)-2-methyl-4-nitrophenylamine.

2. Composition according to Claim 1, **characterized in that** the compound of the formula (I) is present in an amount of 0.01 and 10 per cent by weight.

3. Composition according to Claim 1 or 2, **characterized in that** it has a pH of 3 to 12.

4. Composition according to one of Claims 1 to 3, **characterized in that** it additionally comprises 0.1 to 10 per cent by weight of oxidation dye precursors which require oxidative treatment (developer substances and coupler substances).

5. Composition according to one of Claims 1 to 4, **characterized in that** it additionally comprises 0.01 to 4 per cent by weight of a direct dye from the group consisting of nitro dyes, azo dyes, anthraquinone dyes and triphenylmethane dyes.

6. Composition according to one of Claims 1 to 3 or 5, **characterized in that** it additionally comprises 1 to 5 per cent by weight of a natural or synthetic polymer or of a modified polymer of natural origin, and has a pH of 6 to 9.

7. Method for the colouring of hair, **characterized in that** an amount of a composition according to one of Claims 1 to 3, 5 or 6 sufficient for the colouring is applied to the hair, and, after a contact time of from 15 to 30 minutes, the hair is rinsed with water and dried.

8. Method for the colouring of hair, **characterized in that** an amount of a composition according to one of Claims 1 to 4 sufficient for the colouring is mixed directly prior to use with an oxidizing agent, an amount of the resulting ready-to-use oxidation colorant sufficient for the hair colouring treatment is applied to the hair and, after a contact time of from 10 to 45 minutes at 20 to 50°C, the hair is rinsed with water and dried.

9. The use of a composition according to one of Claims 1 to 6 for the colouring of hair.

## Revendications

1. Composition pour la teinture de fibres, **caractérisée en ce qu'**elle contient au moins un composé choisi dans le groupe constitué par la N-(2-méthoxyéthyl)-4-nitrophénylamine, la N-(2-méthoxyéthyl)-2-méthyl-4-nitrophénylamine, la N-(3-méthoxypropyl)-4-nitrophénylamine et la N-(3-méthoxypropyl)-2-méthyl-4-nitrophénylamine.

2. Composition selon la revendication 1, **caractérisée en ce que** le composé de formule (I) est contenu en une quantité de 0,01 à 10 % en poids.

3. Composition selon la revendication 1 ou 2, **caractérisée en ce qu'**elle présente un pH de 3 à 12.

4. Composition selon l'une quelconque des revendications 1 à 3, **caractérisée en ce qu'**elle contient en outre de 0,1 à 10 % en poids de précurseurs de colorants d'oxydation qui demandent un traitement oxydant (développeurs et coupleurs).

5. Composition selon l'une quelconque des revendications 1 à 4, **caractérisée en ce qu'**elle contient en outre de 0,01 à 4 % en poids d'un colorant direct choisi dans le groupe constitué par les colorants nitrés, les colorants azoïques, les colorants anthraquinoniques et les colorants triphénylméthane.

6. Composition selon l'une quelconque des revendications 1 à 3 ou 5, **caractérisée en ce qu'**elle contient en outre de 1 à 5 % en poids d'un polymère naturel ou synthétique ou d'un polymère modifié d'origine naturelle et présente un pH de 6 à 9.

7. Procédé pour la teinture des cheveux, **caractérisé en ce qu'**on applique sur les cheveux une quantité, suffisante pour la teinture, d'une composition selon l'une quelconque des revendications 1 à 3, 5 ou 6, après un temps d'action de 15 à 30 minutes les cheveux sont rincés à l'eau, puis séchés.

8. Procédé pour la teinture des cheveux, **caractérisé en ce qu'**on mélange une quantité, suffisante pour la teinture, d'une composition selon l'une quelconque des revendications 1 à 4, immédiatement avant l'emploi, avec un oxydant, on applique sur les cheveux une quantité, suffisante pour le traitement de teinture des cheveux, du produit obtenu de teinture par oxydation prêt à l'emploi, et après un temps d'action de 10 à 45 minutes à une température de 20 à 50°C, les cheveux sont rincés à l'eau, puis séchés.

9. Utilisation d'une composition selon l'une quelconque des revendications 1 à 6, pour la teinture des cheveux.
